(19) Europäisches Patentamt
European Patent Office
Office européen des brevets

(11) EP 0 714 913 A1

(12) EUROPÄISCHE PATENTANMELDUNG

(43) Veröffentlichungstag:
**05.06.1996 Patentblatt 1996/23**

(51) Int. Cl.$^6$: **C08B 31/04**, A61L 15/00

(21) Anmeldenummer: **95118221.1**

(22) Anmeldetag: **20.11.1995**

(84) Benannte Vertragsstaaten:
**AT BE CH DE DK ES FR GB GR IE IT LI NL PT SE**

(30) Priorität: **30.11.1994 DE 4442605**

(71) Anmelder: **DEGUSSA AKTIENGESELLSCHAFT**
**D-60311 Frankfurt (DE)**

(72) Erfinder:
- **Oppermann, Wilhelm, Prof. Dr.**
  **D-71083 Herrenberg (DE)**
- **Nebel, Eric**
  **D-70176 Stuttgart (DE)**
- **Dorn, Klaus, Dr.**
  **D-63457 Hanau (DE)**
- **Buchholz, Stefan, Dr.**
  **D-63456 Hanau (DE)**

# (54) Quellbarer Stärkeester, Verfahren zu dessen Herstellung sowie Verwendung

(57) 2.1. Bekannte Superabsorbermaterialien haben entweder eine unzureichende biologische Abbaubarkeit oder, falls diese besteht, ein unzureichendes Quellvermögen oder ein verbesserungsbedürftiges Retentionsvermögen für Wasser.

2.2. Erfindungsgemäß ist ein quellbarer Stärkeester, der zu mehr als 50 Gew.-% aus in Wasser unlöslichen Anteilen besteht und eine Quellkapazität von > 1000 % in einer 1 %igen wässrigen NaCl-Lösung aufweist, jeweils bezogen auf das Gewicht des trockenen Stärkeesters, wobei die Quellkapazität dadurch bestimmt wird, daß man 0,5 g des Stärkeesters in 100 ml einer 1,0 %igen NaCl-Lösung quellen läßt und über einen Papierfilter in einem 90 mm-Büchnertrichter abnutscht bis Luft durch den Filter gesaugt wird bzw. bis keine weitere Testflüssigkeit mehr abgesaugt werden kann und wobei der Stärkeester durch teilweise Veresterung von Stärke oder modifizierter Stärke mit einem Carbonsäureanhydrid oder einer Mischung von Carbonsäureanhydriden und Vernetzung erhältlich ist.

2.3. Superabsorbermaterial mit biologischer Abbaubarkeit.

EP 0 714 913 A1

Printed by Rank Xerox (UK) Business Services
2.11.9/3.4

**Beschreibung**

Die Erfindung betrifft ein Absorptionsmaterial, das überwiegend auf nachwachsenden Rohstoffen basiert und gegenüber den derzeit als Absorbermaterial überwiegend verwendeten Polyacrylaten eine deutlich verbesserte biologische Abbaubarkeit aufweist.

Insbesondere betrifft die Erfindung einen quellbaren Stärkeester, der im Vergleich zu anderen Absorbermaterialien auf Polysaccharidbasis eine hohe Aufnahmekapazität aufweist. Die Erfindung offenbart des weiteren Verfahren zur Herstellung des erfindungsgemäßen Absorptionsmaterials sowie die Verwendung der Produkte.

Die weitaus meisten der heute verwendeten Absorptionsmaterialien, häufig auch als Superabsorber bezeichnet, bestehen aus schwach vernetzten Polyacrylaten und sind somit nur zu einem sehr geringen Teil oder überhaupt nicht abbaubar (s. z. B. Stegman et al., Waste Manage. Res. 11 (1993) 155).

Neben den reinen Polyacrylaten gibt es auch auf Stärke gepfropfte Polyacrylate (DE-A 26 12 846). Der Stärkegehalt dieser Produkte ist mit bis zu 25 % jedoch gering. Bei höheren Stärkegehalten wird eine deutliche Verschlechterung der Absorptionseigenschaften beobachtet. Aufgrund des Polyacrylatgehaltes ist die biologische Abbaubarkeit auch dieser Produkte gering.

Ebenso können bis zu ca. 25 % eines zumindest begrenzt wasserlöslichen Polysaccharides in einen vernetzten Polyacrylatsuperabosorber eingearbeitet werden, indem das Polysaccharid während der Polymerisation des Acrylates in das Reaktionsgemisch eingebracht wird (DE-A 40 29 591, DE-A 40 29 592, DE-A 40 29 593).

US-A 5,079,354 beschreibt ein Absorbermaterial auf Basis von Carboxymethylstärke, also eines Stärkeethers, das durch Umsetzung von Stärke mit Chloressigsäure hergestellt wir. Bei diesem Prozeß wird eine äquivalente Menge Natriumchlorid bezogen auf die eingesetzte Chloressigsäure freigesetzt, was aus ökologischen Gründen unerwünscht ist. Ferner ist bekannt, daß veretherte Polysaccharide bei hohen Substitutionsgraden nur schlecht biologisch abbaubar sind (Mehltretter et al. J. Am. Oil Chem. Soc. 47 (1970) 522).

Aus der DE-A 31 32 976 ist die Verwendung von Stärkesuccinatderivaten als Streckmittel für trockene, feste in Wasser quellbare Absorptionsmittel auf Basis eines ionisch komplexierten anionischen Polyelektrolyten, z. B. Polyacrylsäure/Aluminiumkation-Komplex, bekannt. Dabei werden in der DE-A 31 32 976 eine ganze Reihe von in Frage kommenden Streckmitteln aufgezählt, die auch die Absorptionseigenschaften der sie enthaltenden Mischungen mit z. B. Polyacrylsäure verbessern. So gehören zu den Streckmitteln, welche bevorzugt mit stärker oberflächenbehandelten ionisch komplexierten Polyelektrolyten zu vermischen sind, vor allem Natriumcarboxymethylzellulose, Methylzellulose, fein zerteilter Attapulgitton, Mischungen von Natriumcarboxymethylzellulose mit Attapulgitton oder mit grobem oder feinem Montmorillonitton, kaltwasserdispergierbare Wachsmaisstärke und Stärkesuccinatderivate. In Beispielen zeigen Mischungen aus 40 % walzengetrocknetem Stärkesuccinatderivat und 60 % Polyelektrolyt nur eine geringfügige Verbesserung der Blut-Salz-Druck-Retention des reinen Polyelektrolyten ohne Streckmittel. Für ein reines walzengetrocknetes Stärkesuccinatderivat wird zu Vergleichszwecken ein Wert von 4,0 g/g als Ergebnis des Blut-Salz-Druck-Retention-Tests angegeben. Dieser Wert liegt bei etwa 1/5 des reinen Polyelektrolyten.

Die EP-A 0 603 837 beschreibt die Herstellung von Stärkeestern unter Verwendung von organischen Säureanhydriden. Hierzu läßt man Stärke diverser Herkunft in einem einstufigen, wässrigen Verfahren mit organischen Säureanhydriden der allgemeinen Formel I reagieren,

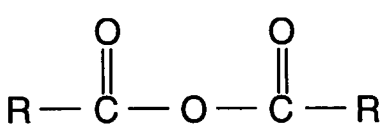

I

worin R alkyl, aryl, alkenyl, alkaryl oder aralkyl mit 1 bis 7 C-Atomen bedeutet, unter bestimmten pH-, Temperatur- und Konzentrationsbedingungen. Zu beispielhaft in der Beschreibung der EP-A 0 603 837 aufgezählten Stärkeestern gehören Stärkeacetat, -propionat, -butyrat, -hexanoat, -benzoat oder auch gemischte Stärkeacetate/propionate. In den Beispielen der EP-A 0 603 837 wird die Verwendung von Propionsäureanhydrid, Acetanhydrid und/oder Buttersäureanhydrid offenbart. Mit dem in der EP-A 0 603 837 beschriebenen Verfahren gelingt es, die Probleme zu umgehen, die sich sonst beim Einsatz größerer Mengen Anhydrid ergeben haben, wie etwa das Quellen oder Gelantinieren der Stärke und Probleme beim Abtrennen der Stärkeester aus der Reaktionsmischung. Das Produkt wird nämlich durch die Umsetzung hydrophob und daher in einfacher Weise abfiltrierbar.

Aus der WO 93/01217 (PCT/EP 92/01553) ist ein Verfahren zur Herstellung von Stärkeestern für klinische, insbesondere parenterale Anwendung bekannt. Zur Herstellung von wasserlöslichen, physiologisch verträglichen Stärkeestern wird Stärke durch Säurehydrolyse oder Enzymhydrolyse in ein Teilhydrolysat mit einem mittleren $M_w$ im Bereich von 10.000 bis 500.000 Dalton überführt, dieses Teilhydrolysat in wässriger Lösung mit dem Anhydrid oder Halogenid

einer aliphatischen Monocarbonsäure mit 2 - 4 Kohlenstoffatomen oder einer aliphatischen Dicarbonsäure mit 3 - 6 Kohlenstoffatomen oder Gemischen davon und einem Alkalisierungsmittel bis zu einer molaren Substitution im Bereich von 0,1 bis 1,0 Mol/Mol acyliert.

Die Stärkeester gemäß der WO 93/01217 sind sehr gut wasserlöslich, was für die parenterale Anwendung zwingend erforderlich ist.

Als Blutplasmaverdicker ist beispielsweise dieser Anmeldung gemäß Beispielen eine durch Umsetzung eines Wachsmaisstärke-Teilhydrolysats mit Essigsäureanhydrid erhältliche Acetylstärke entnehmbar.

Obwohl weitere Stärken und Anhydride in der WO 93/01217 genannt werden, u. a. Anhydride oder Halogenide einer Dicarbonsäure, z. B. Bernsteinsäure oder Maleinsäure, lehrt die in Rede stehende Druckschrift ausschließlich die Herstellung wasserlöslicher, physiologisch verträglicher Stärkeester.

Es hat auch bereits Versuche gegeben, biologisch abbaubare Superabsorber zu schaffen. So kennt man aus der DE-A 42 06 857 ein Absorptionsmittel, das aus einer auf speziellen nachwachsenden Polysaccharid-Rohstoffen basierenden Komponente, einem synthetischen wasserquellbaren Polymeren, das biologisch nicht oder nur zu einem sehr geringen Teil abbaubar ist, einem Matrixmaterial, einem ionischen oder kovalenten Vernetzer und einem Reaktivzusatz besteht. Die auf nachwachsenden Polysaccharid-Rohstoffen basierende Komponente umfaßt z. B. Guar, Carboxymethylguar, Xanthan, Alginate, Gummi Arabicum, Hydroxyethyl- oder Hydroxypropylcellulose, Carboxymethylcellulose und andere Cellulosederivate, Stärke und Stärkederivate wie Carboxymethylstärke. Ferner ist es aus der DE-A 42 06 857 bekannt, daß die aufgeführten Polymeren durch eine Vernetzung modifiziert werden können, um ihre Wasserlöslichkeit zu reduzieren und bessere Quelleigenschaften zu erreichen. Die Vernetzung kann sowohl im gesamten Polymeren stattfinden oder aber auch nur an der Oberfläche der einzelnen Polymerpartikel.

Die Umsetzung der Polymeren kann mit ionischen Vernetzern wie z. B. Calcium-, Aluminium-, Zirkon und Eisen(III)-Verbindungen erfolgen. Ebenso ist die Umsetzung mit polyfunktionellen Carbonsäuren wie Citronensäure, Schleimsäure, Weinsäure, Äpfelsäure, Malonsäure, Bernsteinsäure, Glutarsäure, Adipinsäure, mit Alkoholen wie Polyethylenglykole, Glycerin, Pentaerythrit, Propandiole, Saccharose, mit Kohlensäureestern wie Glykoldiglycidylether, Glykoldi- oder -triglycidylether und Epichlorhydrin, mit Säureanhydriden wie Bernsteinsäureanhydrid und Maleinsäureanhydrid, mit Aldehyden und mehrfunktionellen (aktivierten) Olefinen wie Bis-(acrylamido)-essigsäure und Methylenbisacrylamid möglich. Ebenso kommen natürlich Derivate der genannten Verbindungsklassen in Betracht sowie heterofunktionelle Verbindungen mit verschieden funktionellen Gruppen der oben genannten Verbindungsklassen.

Obwohl die vorgestellten und anhand von Beispielen belegten Systeme auf Basis von Natriumcarboxymethylcellulose in Verbindung mit Natriumpolyacrylat in verschiedenen Tests recht günstige Absorptionseigenschaften aufweisen, ist der Druckschrift in Bezug auf die biologische Abbaubarkeit, mit Ausnahme des Umstandes, daß letztere einfach behauptet wird, nichts Näheres entnehmbar.

Es ist jedoch bekannt, daß Polyacrylate praktisch gar nicht (R. Stegmann et al. Waste Water Res. 11(2) (1993) S. 155) und Carboxymethylcellulose, ein Polysaccaridether nur sehr schlecht biologisch abbaubar ist (4,6 % nach 5 Tagen; M. Seekamp, Textilveredlung 25 (1990) S. 125).

Angesichts des vorstehend diskutierten Standes der Technik ist es Aufgabe der Erfindung gewesen, ein Absorptionsmaterial mit einer den derzeit überwiegend verwendeten Polyarcylaten gegenüber wesentlich verbesserten biologischen Abbaubarkeit anzugeben, sowie ein einfaches Verfahren zu dessen Herstellung zur Verfügung zu stellen.

Gelöst werden diese und weitere im einzelnen nicht näher ausgeführte Aufgaben durch einen quellbaren Stärkeester, der zu mehr als 50 Gew.-% aus in Wasser unlöslichen Anteilen besteht und eine Quellkapazität von > 1000 % in einer 1,0 %igen wässrigen NaCl-Lösung aufweist, jeweils bezogen auf das Gewicht des trockenen Stärkeesters, wobei die Quellkapazität dadurch bestimmt wird, daß man 0,5 g des Stärkeesters in 100 ml einer 1,0 %igen NaCl-Lösung quellen läßt und über einen Papierfilter in einem 90 mm-Büchnertrichter abnutscht bis Luft durch den Filter gesaugt wird bzw. bis keine weitere Testflüssigkeit mehr abgesaugt werden kann, und wobei der Stärkeester durch teilweise Veresterung von Stärke oder modifizierter Stärke mit einem Carbonsäureanhydrid oder einer Mischung von Carbonsäureanhydriden und gleichzeitige oder anschließende Vernetzung erhältlich ist.

Im Rahmen der Erfindung wurde überraschend gefunden, daß bei der Modifizierung kaltwasserlöslicher, d. h. vorgelatinisierter Stärke ein stark quellbares aber zu mehr als 50 % unlösliches Produkte erhältlich ist. Dieses Material ist für den Einsatz als Absorptionsmaterial zu Absorption von Wasser, wäßrigen Lösungen, Dispersionen und Körperflüssigkeiten in Hygiene- und Tierhygiene, insbesondere in Windeln und Inkontinenzprodukten, sowie in Verpackungsmaterialien für Fleisch und Fisch, sowie zur Bodenverbesserung, zum Einsatz in Kulturgefäßen und als Kabelummantelungen geeignet, wobei es gleichzeitig hervorragend biologisch abbaubar ist.

Im Gegensatz zu den in der DE-A 42 06 857 als Komponente A angegebenen Substanzen, deren biologische Abbaubarkeit einfach behauptet wird, weil sie - wie es heißt - auf speziellen nachwachsenden Polysaccharid-Rohstoffen basieren, sind die Superabsorbermaterialien gemäß der vorliegenden Erfindung tatsächlich biologisch abbaubar, wie später eingehender an Modellverbindungen erörtert werden wird.

In bevorzugter erfindungsgemäßer Ausführungsform kennzeichnet sich der Stärkeester der Erfindung durch eine Quellkapazität in 1,0 gew.-%iger wässriger NaCl-Lösung von > 1500 % bezogen auf das Gewicht des trockenen, ungequollenen Stärkeesters.

Bei den erfindungsgemäßen Stärkeestern handelt es sich um Produkte mit einem Substitutionsgrad zwischen 0,2 und 2,0, wobei der Substitutionsgrad die Anzahl der Substituenten pro Glucosering angibt. Bei den Estern kann es sich um Produkte mit nur einer Art von Estergruppe handeln oder um gemischte Ester.

Die Ester werden bevorzugt durch Umsetzung von Stärke mit Säureanhydriden erhalten, wobei es sich bei den Anhydriden um cyclische und/oder offenkettige Anhydride handeln kann. Zu den erfindungsgemäß verwendbaren Anhydriden gehören u. a. Acetanhydrid, Propionsäureanhydrid, Bernsteinsäureanhydrid und/oder Maleinsäureanhydrid. Erfindungsgemäß ist Bernsteinsäureanhydrid bevorzugt.

Aus der Umsetzung von Stärke mit offenkettigen Anhydriden resultieren prinzipiell nichtionische Stärkeester, während aus der Umsetzung von Stärke mit cylischen Anhydriden grundsätzlich ionische, carboxylatgruppentragende Stärkeester resultieren. Um die erfindungsgemäßen quellbaren, aber zu mehr als 50 % unlöslichen Absorptionsmaterialien zu erhalten, ist es ganz besonders bevorzugt, wenn das Produkt freie Carboxyl- bzw. Carboxylatgruppen enthält. Daher ist es von Vorteil, wenn es sich zumindest bei einem Teil des eingesetzten Anhydrides um ein cyclisches Anhydrid handelt.

Bei der Umsetzung kaltwasserlöslicher, d. h. vorgelatinisierter Stärke mit Bernsteinsäureanhydrid wird z. B. ein wasserlösliches Produkt erhalten. Hierbei handelt es sich um ein teilverestertes Produkt, das durch anschließende Vernetzung mit einem geeigneten Vernetzungsmittel in einen erfindungsgemäßen Stärkeester überführbar ist.

DE-A 42 06 857 beschreibt, wie bereits weiter oben ausgeführt, ein Absorptionsmittel, das neben dem biologisch nicht abbaubaren Polyacrylat eine Stärke bzw. ein Stärkederivat enthalten kann. In diesem Zusammenhang wird in DE-A 42 06 857 die Vernetzung von Stärke oder Stärkederivaten wie Carboxymethylstärke (also einem Stärkeether) mit Maleinsäureanhydrid zur Verringerung der Löslichkeit und einer damit verbundenen Verbesserung der Quelleigenschaften beschrieben. Demgegenüber liegt der Erfindung die Erkenntnis zugrunde, daß ohne die Verwendung von biologisch nicht abbaubarem Polyacrylat durch die Veresterung von Stärke oder Stärkederivaten mit Carbonsäureanhydriden und insbesondere mit Bernsteinsäureanhydrid ionische Gruppen in das Polysaccharidgerüst eingeführt werden können, die aufgrund des durch sie erzeugten osmotischen Druckes zu besonders günstigen Absorptions- und Quelleigenschaften führen, und daß dabei eine hervorragende biologische Abbaubarkeit erhalten bleibt.

Zur Erzielung eines vorteilhaften Quellvermögens ist neben der Einführung ionischer Gruppen auch eine Vernetzung notwendig, um das Material in eine zwar quellbare aber wasserunlösliche Form zu überführen.

Beides sowohl Veresterung zur Einführung ionischer Gruppen und Vernetzung geschieht dabei im Rahmen der Erfindung besonders vorteilhaft mit Bernsteinsäureanhydrid oder einer Mischung von wenigstens zwei Anhydriden, von denen eines Bernsteinsäureanhydrid ist, und anschließender Vernetzung von nicht veresterten Hydroxylgruppen der Stärke. Wenn es aber wie gemäß der DE-A 42 06 857 an einer ausreichenden Zahl an ionischen Gruppen im Molekül fehlt, dann ergibt sich ein völlig andersartiges Produkt, das nicht die Quellbarkeit in Verbindung mit dem Retentionsvermögen der quellbaren Stärkeester der Erfindung aufweist.

Als Stärkebasis der erfindungsgemäßen Materialien kann im Prinzip jede native, modifizierte oder subsituierte Stärke eingesetzt werden. Derartige Stärken können aus einer beliebigen pflanzlichen Quelle isoliert worden sein, und umfassen z. B. Kartoffelstärke, Maisstärke, Weizenstärke, Wachsmaisstärke und Stärken mit hohem Amylosegehalt. Stärkemehl kann ebenfalls verwendet werden. Auch verwendet werden können modifizerte Produkte auf Basis einer der oben aufgeführten Stärken wie z. B. säurehydrolysierte Stärke, enzymhydrolysierte Stärke, Dextrine und oxidierte Stärke. Des weiteren können derivatisierte Stärken wie kationische Stärke, anionische Stärke, amphotere Stärke oder nichtionisch modifizierte Stärke wie z. B. Hydroxyethylstärke verwendet werden. Bei den verwendeten Stärken kann es sich um granuläre oder vorgelatinisierte Stärke handeln, wobei die Zerstörung der granulären Struktur thermisch, mechanisch oder chemisch erfolgen kann.

Besonders vorteilhaft für die Erfindung ist der Einsatz kaltwasserlöslicher Stärke. Hierunter versteht man insbesondere vorgelatinierte oder partiell abgebaute Stärke. Hierzu gehört u. a. Aeromyl 115 der Firma Südstärke.

In bevorzugter Ausführungsform ist der erfindungsgemäße quellbare Stärkeester durch Teilveresterung der genannten Stärken und anschließende Vernetzung zugänglich. Dabei kommt grundsätzlich und vorteilhaft jede dem Fachmann geläufige, zu einer ausreichenden Vernetzung geeignete, wenigstens zweifach funktionelle Verbindung in Frage sowie Aldehyde wie z. B. Formaldehyd, der durch Bildung einer Acetalstruktur zu einer Vernetzung führen kann. Beispiele sind ferner Epichlorhydrin, Diepoxide sowie Oligo- und Polyepoxide wie z. B. Kymene H557 der Firma Hercules, $POCl_3$, $PCL_5$, $SbOCl_3$, $SbCl_5$, Methylenbisacrylamid, Cyanurchlorid, Dihalogenalkane, Dichloressigsäure sowie Dianhydride wie z. B. Butantetracarbonsäuredianhydrid und Benzoltetracarbonsäuredianhydrid und Polyanhydride.

Divinylverbindungen, wie beispielsweise Divinylsulfon, sind u. a. bevorzugt. Die Divinylsulfon-Vernetzung kann vorteilhafterweise in wässrigem Medium, bevorzugt in homogener Lösung durchgeführt werden.

Dies eröffnet u. a. auch den Vorteil, das teilveresterte Stärkeprodukt nach der Veresterung mit Carbonsäureanhydrid nicht notwendigerweise isolieren zu müssen.

Weiterhin eignen sich zur Vernetzung ganz besonders bifunktionelle Isocyanat-Verbindungen. Besonders günstig läßt sich beispielsweise Hexamethylendiisocyanat verwenden.

Zur Vernetzung weiterhin geeignet sind auch Di-, Oligo- oder Polyanhydride. Diese können vor oder nach der Umsetzung des Carbonsäureanhydrids mit der Stärke oder der modifizierten Stärke eingesetzt werden. Zu den besonders

bevorzugten Vernetzungsmitteln dieser Art gehört Butantetracarbonsäuredianhydrid, welches bevorzugt zur Behandlung von noch nicht isolierter, mit Bernsteinsäureanhydrid umgesetzter Stärke verwendet wird.

Die Erfindung betrifft auch ein Verfahren zur Herstellung eines Stärkeesters gemäß der Erfindung, bei dem Stärke oder modifizierte Stärke in einer wässrigen Reaktion mit einem Carbonsäureanhydrid oder einer Mischung von Carbonsäureanhydriden bei einem pH-Wert von 7 bis 11 und einer Temperatur von 0 bis 40 °C unter Erhalt einer überwiegend in Wasser löslichen teilveresterten Stärke umgesetzt wird, wobei der pH-Wert durch Zugabe von wässriger Alkali-Lösung mit einer Konzentration von ca. 10 bis 50 Gew.-% im gewünschten Bereich gehalten wird. Das Herstellverfahren ist ferner dadurch gekennzeichnet, daß die teilveresterte Stärke anschließend oder während der Veresterung mit einem wenigstens zwei ungesättigte Gruppen aufweisenden Vernetzungsmittel behandelt wird. Die Durchführung der Vernetzung während der Veresterung bietet sich insbesondere bei der Vernetzung mit Dianhydriden an.

Während der Reaktion des Anhydrides mit der Stärke wird der pH vorzugsweise konstant gehalten. Der pH sollte während der Reaktion zwischen 7 und 11 liegen. Bevorzugt wird ein pH Wert zwischen 8 und 9. Der pH kann im Prinzip durch Zugabe eines beliebigen alkalischen Materials konstant gehalten werden. Besonders nützlich sind Alkali- und Erdalkalihydoxide sowie die Oxide und Carbonate dieser Metalle. Beispielhaft genannt seien Natriumhydroxid, Kaliumhydroxid, Calciumhydroxid, Ammoniumhydroxid, Magnesiumhydroxid und Natriumcarbonat. Bei der Herstellung von Produkten mit einem höheren Substitutionsgrad wird bevorzugt eine Alkalilösung mit einer höheren Konzentration, ca. 10 bis 50 %ig, verwendet um eine unnötige Verdünnung des Reaktionsmediums zu vermeiden.

Die Vernetzung des so erhältlichen teilveresterten Stärkeprodukts ist aufgrund der unterschiedlichen Reaktivität der drei Hydroxylgruppen stark abhängig vom Substitutionsgrad. Das Veresterungsreagenz reagiert bevorzugt mit den räumlich günstig stehenden Hydroxylgruppen der Stärke, welches vorwiegend die primären in 6-Stellung sein werden.

Besonders geeignete Vernetzungsmittel sind die, die zwei ungesättigte Gruppen aufweisen. Hierzu gehören u. a. Divinylverbindungen oder Diisocyanate. Besonders vorteilhafte Vertreter sind Divinylsulfon und Hexamethylendiisocyanat.

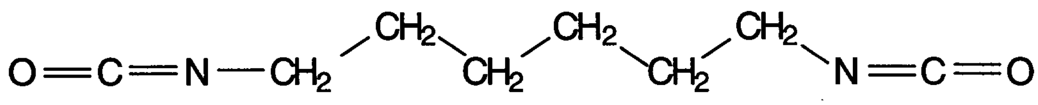

Hexamethylendiisocyanat

$$CH_2{=}CH{-}SO_2{-}CH{=}CH_2$$

Divinylsulfon

Wird mit Divinyl-Verbindungen wie mit Divinylsulfon chemisch vernetzt, so entstehen quellfähige Hydrogele. Dabei reagiert die Vinylsulfonverbindung in einer Art Michael-Addition mit der Hxdroxylgruppe des Polysaccharids unter Ausbildung einer kovalenten Bindung. Zuerst muß die Hydroxylgruppe mit einem basischen Katalysator in das entsprechend reaktivere Anion umgewandelt werden, welches dann an der aktivierten C=C-Doppelbindung angreifen kann:

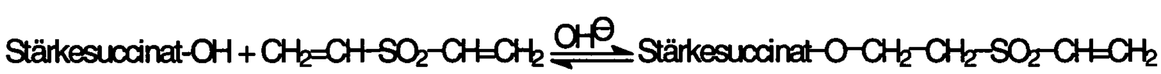


Erfindungsgemäß besteht der Vorteil dieser Reaktion darin, daß in wäßrigem Medium gearbeitet werden kann. Man hat dadurch die Möglichkeit, im Quellungsmittel zu vernetzen, wodurch vorgequollene Gele mit einer definierten Geometrie hergestellt werden können. Die Umsetzung in wäßrigem Medium ist deshalb möglich, da die Hauptmenge des Alkalis von dem Polysaccharid adsorbiert wird, wobei Polysaccharid/Alkai-Komplexe entstehen, deren Reaktivität so hoch ist, daß die Reaktion des Divinylsulfons mit dem Polysaccharid gegenüber der Hydrolysereaktion deutlich dominiert.

Wird mit Diisocyanaten wie mit Hexamethylendiisocyanat vernetzt, so entstehen ebenfalls erfindungsgemäße, quellbare Stärkeester.

Isocyanate haben eine deutlich höhere Reaktivität als Vinylsulfon. So reagiert Phenylisocyanat mit Stärke rasch zu voll substituierten Derivaten. Diese Reaktion wird u. a. zur Stärkecharakterisierung ausgenutzt.

Die hohe Reaktivität wird durch den ausgeprägten ungesättigten Charakter der Isocyanatgruppe erklärt. In einer Additionsreaktion greift das nucleophile Sauerstoffatom der Hydroxylgruppe am elektrophilen Kohlenstoffatom der Isocyanatgruppe an, während das Wasserstoffatom in der Regel von dem Stickstoff gebunden wird. Es entsteht ein Carbamidsäureester (Urethan):

$$\text{Stärkesuccinat}-OH + O{=}C{=}N-R-N{=}C{=}O \xrightleftharpoons{Py} \text{Stärkesuccinat}-O-CO-NH-R-N{=}C{=}O$$

$(R = C_6H_{12})$

Aufgrund der hohen Reaktivität neigen Isocyanate zu Nebenreaktionen. Die Isocyanatgruppe kann praktisch mit allen chemischen Verbindungen, welche ein "aktives" Wasserstoffatom besitzen, reagieren, aber auch mit sich selbst. Mit Wasser bilden sich u. a. N,N'-disubstituierte Harnstoffe, mit Carbonsäuregruppen entstehen Carbamidsäuren, die unter Kohlendioxid-Entwicklung zu substituierten Carbonamiden zerfallen:

$$2R\text{-}N{=}C{=}O + H_2O \rightleftharpoons R\text{-}NH\text{-}CO\text{-}NH\text{-}R + CO_2$$

$$R\text{-}N{=}C{=}O + R_1\text{-}COOH \rightleftharpoons R\text{-}NH\text{-}CO\text{-}O\text{-}CO\text{-}R_1 \rightleftharpoons R\text{-}NH\text{-}CO\text{-}R_1 + CO_2$$

Für Reaktionen von Isocyanaten stehen eine große Anzahl an Katalysatoren zur Verfügung. Für die Umsetzung mit Stärke ist u. a. Pyridin geeignet. Ein weiterer Katalysator ist 1,4 Diaza-(2,2,2)-bicyclooctan, welches besonders gut in der Lage ist, die Reaktion von Isocyanaten mit Hydroxylgruppen zu katalysieren.

Um Nebenreaktionen mit restlichem an der Stärke anhaftendem Wasser zu vermeiden, wird dieses bevorzugt durch azeotropes Abdestillieren entfernt.

Im Gegensatz zur Umsetzung mit Divinylsulfon handelt es sich hier um eine vollständig heterogene Reaktion, bei der das Stärkesuccinat nicht gelöst wird. Dementsprechend ist eine bevorzugte Vernetzung an der Oberfläche der Stärkeesterpartikel zu erwarten.

Um eine optimale Vernetzung im Hinblick auf das Wasseraufnahmevermögen zu erhalten, kann man einzelne Parameter wie Vernetzterkonzentration, Reaktionsdauer und Substitutionsgrad gezielt variieren. Dabei zeigte sich auch, daß Stärkesuccinat, das einer entsprechenden Behandlung in Pyridin ausgesetzt wird, auch ohne Zugabe von Vernetzern (beispielsweise HMDI) zu einem vernetzten Produkt umgesetzt wird. Dabei reagiert wohl eine Hydroxylgruppe mit der Carboxylatgruppe des Succinates unter Abspaltung eines $H_2O$ Moleküls, wobei eine Esterbindung entsteht. Das azeotrope Entfernen des Wassers begünstigt diese Veresterung. Um die Eigenvernetzung des Stärkesuccinats zu verhindern oder zu unterdrücken, kann man auf das azeotrope Abdestillieren verzichten.

Als Lösungsmittel bevorzugt sind jeweils mit 4 Å Molsieb getrocknetes Toluol oder Dimethylformamid (DMF).

Gegenstand der Erfindung ist auch die Verwendung des Stärkeesters in einer Menge von 100 Gew.-Teilen zusammen mit 0,7 - 70 Gew.-Teilen eines Antiblockingmittels auf Basis natürlicher oder synthetischer, bevorzugt hydrophiler Fasern oder Materialien mit großer Oberfläche als Superabsorber. Bevorzugt ist die Verwendung des Stärkeesters als Superabsorber zusammen mit 1 bis 5 Gew.-Teilen Kieselsäure oder Cellulosefasern als Antiblockingmittel.

Weitere Anwendung findet der Stärkeester der Erfindung als Absorptionsmaterial zur Absorption von Wasser, wäßrigen Lösungen, Dispersionen und Körperflüssigkeiten in Hygiene- und Tierhygiene, insbesondere in Windeln, Tampons und Inkontinenzprbdukten sowie in Verpackungsmaterialien für Fleisch und Fisch, als Absorptionsmaterial zur Absorption von Wasser und wäßrigen Lösungen in Kulturgefäßen und zur Bodenverbesserung bder als Absorptionsmaterial zur Absorption von Wasser und wäßrigen Lösungen in Kabelummantelungen.

Die erfindungsgemäßen Materialien sind biologisch abbaubar. Für quellbare, aber wasserunlösliche Produkte gibt es z. Z. noch keinen allgemein anerkannten Test zur Bestimmung der biologischen Abbaubarkeit. Um dennoch eine zumindest qualitative Aussage treffen zu können, wurde die biologische Abbaubarkeit des unvernetzten Stärkesuccinates im Zahn-Wellens-Test untersucht. Stärkesuccinat mit einem DS(th.) = 1,0 (DS(NaOH) = 0,6) wird innerhalb von 28 Tagen zu 90 % biologisch abgebaut. Die niedermolekulare Modellverbindungen, Dextrinsuccinat mit einem DS(th.) = 1,0 ((DS(NMR1) = 0,5; DS(NMR6) = 0,7) bzw. Dextrinsuccinat mit einem DS(th.) = 2,0 ((DS(NMR1) = 0,9; DS(NMR6) = 1,5) werden innerhalb von 28 Tagen zu jeweils 89 % abgebaut.

Zum Vergleich: Für die bei der Synthese des Stärkesuccinates eingesetzte unsubstituierte Stärke wird nach 26 Tagen ein Abbau von 83 % gefunden. Somit wird die biologische Abbaubarkeit der Stärke durch die Succinatreste auch bei hohen Substitutionsgraden im Rahmen der Meßgenauigkeit nicht beeinträchtigt.

Für Carboxymethylstärke, einem Stärkeether mit einem theoretischen DS von 1,0 wird hingegen nach 21 Tagen nur ein biologischer Abbau von 13 % gefunden, was die literaturbekannte schlechte biologische Abbaubarkeit von Stärkeethern belegt (Mehltretter et al. J. Am. Oil Chem. Soc. 47 (1970) 522).

**Bestimmung der Substitutionsausbeute über der NaOH-Verbrauch während der Synthese**

Bei der Umsetzung von Stärke mit Bernsteinsäureanhydrid (BSA)in wäßriger Lösung wird pro mol Bernsteinsäureanhydrid ein mol NaOH verbraucht. Findet keine Addition des Bernsteinsäureanhydrides an die Stärke sondern eine Hydrolyse zum Dinatriumsuccinat statt, so werden, bei pH-Konstanthaltung, pro Mol MSA 2 Mol NaOH verbraucht. Wenn am Ende der Reaktion kein nicht reagiertes Anhydrid mehr vorliegt, läßt sich der Substitutionsgrad nach

$$DS = 1 - \frac{(\text{Verbrauch an NaOH in Mol - eingesetzte Menge MSA in Mol})}{(\text{eingesetzte Menge MSA in Mol})}$$

berechnen. Der so ermittelte Substitutionsgrad wird im folgenden mit DS(NaOH) bezeichnet.

**Bestimmung der Subsitutionsausbeute mittels NMR**

Das Verhältnis des Integrals für die Signale der gebundenen Bernsteinsäure zu dem Intergal des glycosidischen H-Atome läßt sich der Substitutionsgrad errechnen. Der so ermittelte Wert wird mit DS(NMR1) abgekürzt. Das Integral des Signals der gebundenen Bernsteinsäure kann aber auch dem der sechs H-Atome im Ring in Beziehung gesetzt werden. Der so berechnete Substitutionsgrad wird als DS(NMR6) bezeichnet. Die Bestimmung der Integrale breiter NMR-Signal ist relativ ungenau. Dementsprechend können DS(NMR1) und DS(NMR6) erheblich, um bis zu ca. 30 %, voneinander abweichen.

**Methode zur Bestimmung des Rückhaltevermögens (Retentionsvermögens)**

Zur Bestimmung des Quellvermögens definiert man ein Lösungsmittelaufnahmevermögen, das angibt, wieviel Gramm Lösungsmittel von einem Gramm lösungsmittelfreiem Gel bei der Quellung aufgenommen und zurückgehalten wird. Einfachste Methode ist die Messung der freien Quellkapazität (Absorption). Dazu wird das Gel in einem Überschuß an Lösungsmittel gequollen. Nach einer bestimmten Zeit werden die gequollenen Partikel durch Filtration von nicht absorbiertem Lösungsmittel getrennt und gewogen. Problem dieser Methode ist, daß der Filter leicht verstopft, wodurch unrealistisch hohe Werte erhalten werden. Umso stärker ein Gel quellbar ist, desto weicher und labiler wird es. Die Abweichung von der tatsächlichen Quellung wird in diesem Fall immer größer.

Um nun einen Wert für die effektive Quellkapazität (Retention) zu bekommen, ist es nötig, sowohl zwischen den Partikeln als auch auf der Oberfläche haftende Flüssigkeit zu entfernen. Dies wird durch Absaugen mit einer Vakuumpumpe versucht (Quellkapazitätstest). Der Erfolg dieser Methode ist aber stark von der Partikelgröße und auch von deren mechanischer Stabilität abhängig.

Das überschüssige Lösungsmittel kann man auch durch Zentrifugation entfernen. In dem sogenannten "Teebeuteltest" wird eine genau eingewogene Probenmenge in einen Teebeutel eingeschweißt und für eine bestimmte Zeit in das Lösungsmittel getaucht, um sich vollzusaugen. Danach wird bei einer bestimmten Umdrehungsgeschwindigkeit zentrifugiert und gewogen.

Alternativ gibt es die Möglichkeit, das Gel unter einem bestimmten Druck quellen zu lassen. Dazu beschwert man das Gel mit einem definierten Gewicht und läßt die Flüssigkeit durch eine Fritte von unten an das Gel herantreten. Die nach diesen Methoden gemessene Retention liefert im Vergleich zur Absorption realistischere Werte.

Eine Methode, bei der das reine Gelvolumen von Partikeln im gequollenen Zustand ermittelt werden kann, ist der Dextranblau-Test. Eine abgewogene Probe des Gels wird für eine bestimmte Zeit in einer Lösung gequollen, die eine entsprechende Menge Salz und den Farbstoff Blue Dextran enthält. Aufgrund seines hohen Molekulargewichts ist der Farbstoff nicht in der Lage, in das Gel zu diffundieren. Beim Quellprozeß reichert sich der Farbstoff in der überstehenden Lösung an. Die Konzentrationszunahme kann dann UV-spektroskopisch mit Hilfe des Lambert Beerschen Gesetz bestimmt werden. Die fehlende Lösungsmittelmenge ist die Menge, die vom Gel aufgenommen wird.

**Messung der Partikelfließgrenze**

In einem trockenen 150 ml Becherglas werden ungefähr 0,5 g trockenes Produkt eingewogen. Danach tropft man langsam die zu testende Meßflüssigkeit (demineralisiertes Wasser oder 1 %ige NaCl-Lösung) so lange zu, bis das Gel nicht mehr in der Lage ist, die Flüssigkeit spontan einzusaugen und zu immobilisieren. Ab diesem Punkt beginnt die Partikelmasse beim Schwenken des Becherglases zu fließen. Es wird das Gewicht der zugegebenen Meßflüssigkeit gemessen und in g absorbierte Meßflüssigkeit / g SAP umgerechnet.

**Quellkapazitätstest**

In einem trockenen 300 ml Becherglas werden ungefähr 0,5 g trockenes Produkt eingewogen. Danach wird 100 ml Testflüssigkeit zugegeben und für eine bestimmte Zeit gequollen. Unter Ausschwenken wird die Probe in einen mit einem Papierfilter (Schwarzband-Rundfilter der Firma Schleicher und Schüll) und einer Saugflasche ausgerüsteten 90 mm Büchnertrichter geschüttet. Die im Becherglas verbliebenen Gelpartikel werden mit 25 ml Testflüssigkeit in den Büchnertrichter gespült. Dann wird mit einer Pumpe (Membranpumpe der Firma Vacubrand) so lange ein Unterdruck von maximal 30 mbar angelegt bis Luft durch den Filter gesaugt wird. Bei Proben, die stark zum Verschleimen neigen, gelingt es nicht, Luft durch den Filter zu ziehen. Hier wird dann so lange gesaugt, bis so gut wie keine Testflüssigkeit mehr in die Saugflasche tropft. Gemessen wurde das Gewicht der im Filter verbliebenen Gelpartikel und in g absorbierte Meßflüssigkeit / g SAP umgerechnet.

**Dextranblau-Test**

a) Herstellung der Dextranblau-Lösung

0,075 g Dextranblau werden unter leichtem Erwärmen in 250 g 1 %iger NaCl-Lösung gelöst. Diese 0,03 %igen Lösungen besitzen ein UV/VIS Spektrum, das zwei Maxima aufweist. Zur Messung wurde das Maximum bei 618 nm (E = 0,2800) verwendet.

b) Messung

In zwei verschließbare 25 ml Glasbehälter werden jeweils 0,1 g des zu testenden Materials sehr genau eingewogen. Die eine Probe wird mit 15 g Dextranblau-Lösung versetzt und für eine bestimmte Zeit gequollen. Parallel dazu wird die zweite Probe mit 15 g 1 %iger NaCl-Lösung versetzt und für die gleiche Zeit gequollen. Sie dient als Referenzprobe. Alle Gewichtsmessungen wurden mit einer Waage der Firma Satorius durchgeführt und auf 0,0001 g genau bestimmt. Um eine möglichst homogene Quellung zu erreichen, werden die Proben von Zeit zu Zeit von Hand geschüttelt.

Die Messungen erfolgten nach 4 bzw. 24 Stunden. Dazu wird ungefähr 3 ml überstehende Lösung mit einer Pipette in eine Küvette gefüllt. Damit keine eventuell vorhandenen Gelpartikel die Messung stören, wird die Lösung vor der Messung für 5 Minuten zentrifugiert.

Die Messungen erfolgten an einem UV/VIS Spektrometer Lambda 2 der Firma Perkin Elmer. Dabei wird die Extinktion der Dextranblau-Lösung ($E_{BD\text{-}Lsg.}$), der mit Dextranblau-Lösung gequollenen Probe ($E_{BD\text{-}SAP}$) und der mit 1 %iger NaCl-Lösung gequollenen Probe ($E_{REF}$) genau gemessen. Der Nullabgleich erfolgt mit der 1 %igen NaCl-Lösung.

Der Quellungsgrad q, in g absorbierte Meßflüssigkeit / g SAP, errechnet sich nach folgender Gleichung:

$$q=\frac{Einwaage_{BD\text{-}Lsg.}}{Einwaage_{SAP}}\left(1-\frac{E_{BD\text{-}Lsg.}}{E_{BD\text{-}SAP}-E_{REF}}\right)$$

**Bestimmung der löslichen Anteile**

Zur Bestimmung der löslichen Anteile wird 1 g des zu charakterisierenden Produktes in 100 ml entmineralisiertes Wasser gegeben und es wird 24 Stunden bei Raumtemperatur gerührt. Die entstandenen Gelpartikel werden abzentrifugiert und es wird eine Probe von ca. 10 ml von der überstehenden Lösung abgenommen, gewogen, einrotiert, der Rückstand wird gewogen und die gemessene Menge wird auf die Gesamteinwaage an Absorbermaterial zurückgerechnet.

**Biologischer Abbau nach Zahn-Wellens**

Der Abbautest nach Zahn und Wellens ist ein Hilfsmittel, die biologische Abbaubarkeit einer Substanz bzw. eines Abwassers zu beurteilen. Er ist in quantitativer Weise nur auf wasserlösliche Substanzen anwendbar. Für nicht vollständig lösbare Substanzen gibt er nur einen qualitativen Hinweis, ob diese Substanzen prinzipiell einem biologischen Abbau zugänglich sind oder nicht.

In einem hohen 3 l-Becherglas werden ca. 2 l Biomassesuspension gerührt und über Glasfritten belüftet. Es werden 385 mg $NH_4Cl$ und 89 mg $NaH_2PO_4H_2O$ eingewogen, mit der berechneten Polymermenge versetzt und mit kaltem Leitungswasser auf ca. 2 l aufgefüllt. Der Belebtschlamm aus einer kommunalen Kläranlage wird 30 - 60 in absetzen lassen, so daß er ca. um die Hälfte eindickt. Das überstehende Wasser wird abdekantiert und der Schlamm unter Rühren und Belüften aufbewahrt. Jeweils ein Teil des Schlammes wird davon abgenommen und bei 200 Upm 5 min zentrifugiert. 24 g des zentrifugierten Schlammes auf einen 2 l-Ansatz ergeben einen Trockensubstanzgehalt von 1 g/l (± 200 %). Vor

der Zugabe des Schlammes zur Polymerlösung wird der pH auf 6,5 - 7,0 eingestellt und eine Probe auf den CSB-Gehalt hin analysiert. Nachdem sich der Belebtschlamm fein verteilt hat, kann erneut eine Probe entnommen werden. Die Höhe der Flüssigkeit im Becherglas wird dann markiert. Zum Vergleich wird ein Test angesetzt, der nur Nährsalze und Belebtschlamm, jedoch kein Polymer enthält. Dieser Ansatz dient zur Ermittlung des durch den Belebtschlamm verursachten CSB. Mit einem Gummiwischer wird der am Rand des Becherglases abgesetzte Bioschlamm täglich zurück in die Lösung gewischt, der pH-Wert erneut eingestellt und verdunstetes Wasser durch entmineralisiertes Wasser ersetzt. Zur Bestimmung der am Bioschlamm absorbierten Substanz werden zwei Proben auf ihren CSB-Gehalt analysiert, eine unfiltrierte Probe wird direkt aus dem gut durchmischten Reaktiongefäß entnommen und gemessen. Für die zu filtrierende Probe wird ein aliquoter Teil, zum Beispiel 40 ml, entnommen, absetzen lassen und über ein Millipor 2,5 µ Filter (z. B. Millipor Millex GS) filtriert. Die klare Lösung wird ebenfalls auf ihren CSB-Gehalt analysiert.

Berechnung

Um CSB-Gehalt der Substanz zu ermitteln, der an der Biomassen adsorbiert ist, wird vom CSB in der Probelösung mit Biomasse der CSB-Wert der filtrierten Probe und der Blindwert mit Biomasse abgezogen.

P1 unfiltrierte Probelösung mit Schlamm
P2 filtrierte Probelösung
Bld unfiltrierter Blindwert
ad adsorbierte Substanz

$$P1 - P2 - Bls = ad$$

Wird von der filtrierten Probe P2 der filtrierte Blindwert abgezogen, so erhält man die gelöste Substanz.

B1 filtrierter Blindwert
S gelöste Substanz

$$P2 - Bl = S$$

Adsorbierte und gelöste Substanz zusammen ergeben den Anfangsgehalt an abzubauendem Stoff bzw. den Gehalt zur Zeit. Der biologische Abbau η berechnet sich

A Anfangswert
η biologischer Abbau

$$\eta = \frac{A-(ad+s)}{A} 100$$

**Beispiele**

**Beispiel 1 - Synthese von Stärkesuccinat**

50 g (= 0,278 mol) Aeromyl 115 (physikalisch modifizierte, kaltwasserlösliche Stärke der Firma Südstärke; Restfeuchte = 11,9 %) werden in 400 ml Wasser gelöst. Der pH wird mit 3 N NaOH auf 9 eingestellt und während der Reaktion konstant gehalten. Bei einer Reaktionstemperatur von 25 °C werden 31,5 g (= 0,315 mol) festes Bernsteinsäureanhydrid über einen Zeitraum von 2 h zugegeben. Es wird weitere 3 h bei 25 °C gerührt und anschließend wird das Produkt durch Zugabe von Aceton ausgefällt. Das Produkt wird abfiltriert und bei RT im Vakuumtrockenschrank getrocknet. Die Substitutionsausbeute beträgt 81 % und der Substitutionsgrad 0,91.

**Beispiele 2 - 4 - Synthese von Stärkesuccinaten**

Wie in Beispiel 1, mit dem Unterschied, daß durch Variation der Ausgangsmengen Stärkesuccinate mit den Substitutionsgraden 0,81, 0,72 und 0,47 hergestellt werden.

Die eingesetzten Stärkesuccinate mit den Substitutionsgraden 0,47, 0,91 und 0,72 sind relativ gut in Wasser löslich. Das Stärkesuccinat mit dem Substitutionsgrad 0,81 löste sich dagegen erst nach einiger Zeit.

**Beispiel 5 - Vernetzung mit Hexamethylendiisocyanat**

Verfahren 1:

3 bzw. 5 g Stärkesuccinat werden in über KOH getrocknetem Pyridin suspendiert. Das an der Stärke anhaftende Wasser wird durch azeotrope Destillation entfernt. Die entfernte Menge wird durch neues trockenes Pyridin ersetzt. Danach tropft man HMDI unter Rühren zügig zu, erhitzt auf 100 - 110 °C für 6 Stunden und läßt für 10 - 12 Stunden bei Raumtemperatur rühren. Das Produkt wird durch Zugabe von 40 ml 96 %igem Ethanol ausgefällt. Damit das Ethanol mit eventuell noch nicht umgesetzten HMDI abreagieren kann, wird noch für weitere 10 Minuten gerührt. Mit Hilfe einer Glasfritte wird abfiltriert und zweimal mit 20 ml Ethanol gewaschen. Schließlich wird das Produkt an Luft getrocknet.

Verfahren 2:

Wie Verfahren 2 mit dem Unterschied, daß das entfernte Pyridin nicht mehr ersetzt wird.

Verfahren 3:

5 g Stärkesuccinat werden in 35 ml trockenem Pyridin suspendiert. Es wird für 2 Stunden unter Rühren auf 110 °C erhitzt und nach weiteren 2 Stunden ohne Rühren bei Raumtemperatur wie unter Verfahren 2 beschrieben aufgearbeitet.

Verfahren 4:

3 g Stärkesuccinat werden wiederum in 50 ml des jeweiligen Lösungsmittels suspendiert. Anschließend wird 0,025 g 1,4-Diaza-(2,2,2)-bicyclooctan und die entsprechende Menge HMDI zugegeben. Die Reaktionsmischung wird für 3 Stunden unter Rückfluß erhitzt. Im Falle des Toluols entspricht dies einer Temperatur von ca. 110 °C, bei DMF ca. 120 °C. Die Aufarbeitung erfolgt am nächsten Tag (nach ca. 16 Stunden) wie unter Verfahren 2 beschrieben.

**Beispiel 6 - Synthese von vernetztem Stärkesuccinat mit Butantetracarbonsäuredianhydrid als Vernetzer**

50 g (= 0,272 mol) Aeromyl (physikalisch modifizierte, kaltwasserlösliche Stärke der Firma Südstärke; Restfeuchte = 11,8 %) werden in 400 ml Wasser gelöst. Der pH wird mit 3 N NaOH auf 8 eingestellt und während der Reaktion konstant gehalten. Bei einer Reaktionstemperatur von 0 °C werden 24,5 g (0,245 mol) Bernsteinsäureanhydrid über einen Zeitraum von 2 Stunden zugegeben. Nach einer weiteren Stunde werden in kleinen Portionen 2,69 g (= 0,0136 mol) Butantetracarbonsäuredianhydrid zugegeben und es wird 2 weitere Stunden gerührt. Anschließend wird der Ansatz einrotiert und im Vakuumtrockenschrank nachgetrocknet. Quellkapazität für 1 %ige NaCl-Lösung = 42,5 g/g
Die Versuchsergebnisse sind nachfolgend tabellarisch zusammengefaßt (Tabelle 1):

Tabelle 1

| Nr. | Substitutionsgrad | Vernetzermenge [mmol/g] | Reaktionsbedingungen | Abdestillierte Menge [in ml] | Ausbeute [in g/g] | Partikelfließgrenze [in g/g] | | Quellkapazitätstest [in g/g] | | Dextranblau-Test [in g/g] |
|---|---|---|---|---|---|---|---|---|---|---|
| | | | | | | $H_2O$ | 1 %ige NaCl | $H_2O$ | 1 %ige NaCl | |
| 3 | 0,72 | 1,9 HMDI | Verfahren 1a) | 40 | 90 | 35 | 21 | 52 | 17,5 | - |
| 8 | 0,72 | 1,9 HMDI | Verfahren 1b) | 70 | 99 | 46 | 19,5 | 60 | 18,6 | 19,6 |
| 9 | 0,72 | 0,94 HMDI | Verfahren 1a) | 25 | 80 | 23 | 19 | 63 | 19,6 | 9,9 |
| 10 | 0,72 | 0,67 HMDI | Verfahren 1a) | 40 | 97 | 21 | 20 | 63 | 20,6 | 18,9 |
| 11 | 0,72 | 0,31 HMDI | Verfahren 1a) | 10 | 90 | 31 | 18 | 58 | 17 | 17,4 |
| 13 | 0,91 | 0,07 HMDI | Verfahren 2 | 35 | 87 | 25 | 13 | 120,4 | 27,6 | 23,9 |
| 14 | 0,91 | 0,07 HMDI | 1) b) | 35 | 106 | 11 | 6 | 27,4 | 12 | 12 |
| 16 | 0,47 | 0,07 HMDI | Verfahren 2 b) | 35 | 112 | - | 14 | 48,2 | 15 | 12,6 |
| 17 | 0,91 | 0,07 HMDI | 2) b) | 35 | 100 | - | 8 | nicht meßbar | | 33,4 |
| 18 | 0,91 | 0,19 HMDI | Verfahren 2 b) | 35 | 118 | 29 | 13 | 99,6 | 23,2 | 19,5 |
| 21 | 0,91 | 0 | Verfahren 2 b) | 35 | 100 | - | 8 | 113,1 | 25,1 | 26,3 |
| 22 | 0,91 | 0,04 HMDI | Verfahren 2 b) | 40 | 106 | - | 10 | 59,4 | 17,5 | 18,9 |
| 23 | 0,91 | 0,07 HMDI | 3) | - | 123 | 14 | 10 | 63,2 | 30,8 | 29,4 |
| 24 | 0,72 | 0,9 HMDI | 4) | - | 106 | 27 | 16 | nicht meßbar | | 35,3 |
| 25 | 0,91 | 0,12 HMDI | Verfahren 4 (Toluol) | - | 103 | - | - | 32,9 | 31,8 | 31,8 |
| 26 | 0,72 | 0,9 HMDI | 5) | - | 99 | 27 | 22 | 91,9 | 38,3 | 30,5 |

Tabelle 1 - Fortsetzung:

| Nr. | Substitutionsgrad | Vernetzermenge [mmol/g] | Reaktionsbedingungen | Abdestillierte Menge [in ml] | Ausbeute [in g/g] | Partikelfließgrenze [in g/g] | | Quellkapazitätstest [in g/g] | | Dextranblau-Test [in g/g] |
|---|---|---|---|---|---|---|---|---|---|---|
| | | | | | | $H_2O$ | 1 %ige NaCl | $H_2O$ | 1 %ige NaCl | |
| 27 | 0,91 | 0,12 HMDI | Verfahren 4 (DMF) | - | 116 | - | - | 75,4 | 16,9 | 21,3 |
| 28 | 0,91 | 0,07 HMDI | 6) (DMF) | - | 116 | - | - | 61 | 31 | 38,1 |
| 29 | 0,47 | 0 | Verfahren 3 | - | 112 | 16 | 8 | 105,8 | 22,3 | 24,5 |
| 30 | 0,91 | 0 | Verfahren 3 | - | 103 | 15 | 9 | 80,4 | 34,9 | 40,1 |
| 31 | 0,81 | 0 | Verfahren 3 | - | 99 | 21,6 | 13,7 | 60,7 | 19,9 | 18,7 |

1) 24 Stunden unter Rückfluß, dann nach Verfahren 2.

2) 3 Stunden unter Rückfluß, dann 35 ml abdestilliert und schließlich bei Raumtemperatur 17 Stunden gerührt.

3) Sofortige Zugabe von HMDI, dann 3 Stunden bei 100 °C.

4) Sofortige Zugabe von HMDI, dann 1 Stunde bei 110 °C.

5) 1 Stunde bei 85 °C vor Zugabe des HMDI, danach 1 Stunde bei 85 °C.

6) Sofortige Zugabe von HMDI, dann 2 Stunden bei 120 °C

a) 3 g Ansatz mit 45 ml Lösungsmittel

b) 5 g Ansatz mit 75 ml Lösungsmittel

**Patentansprüche**

1. Quellbarer Stärkeester, der zu mehr als 50 Gew.-% aus in Wasser unlöslichen Anteilen besteht und eine Quellkapazität von > 1000 % in einer 1 %igen wässrigen NaCl-Lösung aufweist, jeweils bezogen auf das Gewicht des trockenen Stärkeesters, wobei die Quellkapazität dadurch bestimmt wird, daß man 0,5 g des Stärkeesters in 100 ml einer 1,0 %igen NaCl-Lösung quellen läßt und über einen Papierfilter in einem 90 mm-Büchnertrichter abnutscht bis Luft durch den Filter gesaugt wird bzw. bis keine weitere Testflüssigkeit mehr abgesaugt werden kann und wobei der Stärkeester durch teilweise Veresterung von Stärke oder modifizierter Stärke mit einem Carbonsäureanhydrid oder einer Mischung von Carbonsäureanhydriden und Vernetzung erhältlich ist.

2. Stärkeester nach Anspruch 1,
**gekennzeichnet durch**
eine Quellkapazität in 1,0 gew.-%ige wässrige NaCl-Lösung von > 1500 % bezogen auf das Gewicht des trockenen, ungequollenen Stärkeesters.

3. Stärkeester nach Anspruch 1 oder 2,
**dadurch gekennzeichnet,**
daß er durch Veresterung von Stärke oder modifizierter Stärke mit Bernsteinsäureanhydrid oder einer Bernsteinsäureanhydrid aufweisenden Mischung von Carbonsäureanhydriden und anschließende Vernetzung erhältlich ist.

4. Stärkeester nach einem der vorhergehenden Ansprüche,
**dadurch gekennzeichnet,**
daß er durch Veresterung und anschließende Vernetzung mit Divinylsulfon erhältlich ist.

5. Stärkeester nach einem der Ansprüche 1 - 3,
**dadurch gekennzeichnet,**
daß er durch Veresterung und anschließende Vernetzung mit Hexamethylendiisocyanat erhältlich ist.

6. Stärkeester nach einem der Ansprüche 1 - 3,
**dadurch gekennzeichnet,**
daß er durch Veresterung mit Bernsteinsäureanhydrid oder einer Bernsteinsäureanhydrid aufweisenden Mischung und Vernetzung mit einem Di-, Oligo- oder Polyanhydrid erhältlich ist.

7. Stärkeester nach Anspruch 6,
**dadurch gekennzeichnet,**
daß er durch Veresterung mit Bernsteinsäureanhydrid oder einer Bernsteinsäureanhydrid aufweisenden Mischung und Vernetzung mit Butantetracarbonsäuredianhydrid erhältlich ist.

8. Stärkeester nach einem der vorhergehenden Ansprüche,
**dadurch gekennzeichnet,**
daß er durch teilweise Veresterung im wässrigen Milieu bei einem pH von 7 bis 11 und einer Temperatur von 0 bis 40 °C, wobei der pH-Wert während der Umsetzung durch Zusatz von Alkali im gewünschten Bereich gehalten wird und dessen Vernetzung vor oder nach Isolierung des teilveresterten Stärkeproduktes erhältlich ist.

9. Verfahren zur Herstellung eines Stärkeesters gemäß einem der Ansprüche 1 - 8, bei dem Stärke oder modifizierte Stärke in einer wässrigen Reaktion mit einem Carbonsäureanhydrid oder einer Mischung von Carbonsäureanhydriden bei einem pH-Wert von 7 bis 11 und einer Temperatur von 0 bis 40 °C unter Erhalt einer überwiegend in Wasser löslichen teilveresterten Stärke umgesetzt wird, wobei der pH-Wert durch Zugabe von wässriger Alkali-Lösung mit einer Konzentration von ca. 10 bis 50 Gew.-% im gewünschten Bereich gehalten wird,
**dadurch gekennzeichnet,**
daß die teilveresterte Stärke anschließend mit einem wenigstens zwei ungesättigte Gruppen aufweisenden Vernetzungsmittel behandelt wird.

10. Verfahren nach Anspruch 9,
**dadurch gekennzeichnet,**
daß als Vernetzer Divinylsulfon in homogener wäßriger Lösung eingesetzt wird.

11. Verfahren nach Anspruch 10,
**dadurch gekennzeichnet,**
daß ohne vorherige Isolierung der teilveresterten Stärke vernetzt wird.

12. Verfahren nach Anspruch 9,
**dadurch gekennzeichnet,**
daß als Vernetzungsmittel Hexamethylendiisocyanat in inhomogener Suspension in einem organischen Lösungsmittel eingesetzt wird.

13. Verfahren nach Anspruch 9,
**dadurch gekennzeichnet,**
daß als Vernetzungsmittel ein Di-, Oligo- oder Polyanhydrid eingesetzt wird.

14. Verfahren nach Anspruch 13,
**dadurch gekennzeichnet,**
daß als Vernetzungsmittel Butantetracarbonsäuredianhydrid eingesetzt wird.

15. Verfahren nach einem der Ansprüche 9 - 14,
**dadurch gekennzeichnet,**
daß als Carbonsäureanhydrid Bernsteinsäureanhydrid verwendet wird oder eine Mischung von Anhydriden, die Bernsteinsäureanhydrid aufweist.

16. Verwendung des Stärkeesters gemäß einem der Ansprüche 1 bis 8 in einer Menge von 100 Gew.-Teilen zusammen mit 0,7 - 70 Gew.-Teilen eines Antiblockingmittels auf Basis natürlicher oder synthetischer, bevorzugt hydrophiler Fasern oder Materialien mit großer Oberfläche als Superabsorber.

17. Verwendung des Stärkeesters gemäß einem der Ansprüche 1 bis 8 als Superabsorber zusammen mit 1 bis 5 Gew.-Teilen Kieselsäure oder Cellulosefasern als Antiblockingmittel.

18. Verwendung des Stärkeesters gemäß einem der Ansprüche 1 bis 8 als Absorptionsmaterial zur Absorption von Wasser, wäßrigen Lösungen, Dispersionen und Körperflüssigkeiten in Hygiene- und Tierhygiene, insbesondere in Windeln, Tampons und Inkontinenzprodukten sowie in Verpackungsmaterialien für Fleisch und Fisch.

19. Verwendung des Stärkeesters gemäß einem der Ansprüche 1 bis 8 als Absorptionsmaterial zur Absorption von Wasser und wäßrigen Lösungen in Kulturgefäßen und zur Bodenverbesserung.

20. Verwendung des Stärkeesters gemäß einem der Ansprüche 1 bis 8 als Absorptionsmaterial zur Absorption von Wasser und wäßrigen Lösungen in Kabelummantelungen.

Europäisches Patentamt

# EUROPÄISCHER RECHERCHENBERICHT

Nummer der Anmeldung
EP 95 11 8221

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | DE-A-25 33 005 (INTERNATIONAL PAPER CO.)<br>* Seite 8, Zeile 22 - Zeile 26 *<br>* Seite 10, Zeile 6 - Zeile 15 *<br>--- | 1-20 | C08B31/04<br>A61L15/00 |
| Y | US-A-2 461 139 (CARLYLE G. CALDWELL)<br>* Spalte 4, Zeile 1-10; Beispiel 4 *<br>--- | 1-20 | |
| Y | DE-B-11 77 129 (W.A. SCHOLTEN 'S CHEMISCHE FABRIEKEN)<br>* Spalte 3, Zeile 60 - Spalte 4, Zeile 18 *<br>--- | 1-20 | |
| A | DATABASE WPI<br>Week 8201<br>Derwent Publications Ltd., London, GB;<br>AN 826E<br>& JP-A-56 155 203 (KURARAY KK) ,<br>1.Dezember 1981<br>* Zusammenfassung *<br>--- | | |
| A | CH-A-631 085 (UNILEVER)<br>----- | | RECHERCHIERTE SACHGEBIETE (Int.Cl.6)<br>C08B<br>A61L |

Der vorliegende Recherchenbericht wurde für alle Patentansprüche erstellt

| Recherchenort | Abschlußdatum der Recherche | Prüfer |
|---|---|---|
| DEN HAAG | 18.März 1996 | Lensen, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)